# EUROPÄISCHE PATENTSCHRIFT

(11) **EP 1 986 613 B1**
(45) Veröffentlichungstag und Bekanntmachung des Hinweises auf die Patenterteilung: **11.07.2012**
(21) Anmeldenummer: 07721990.5
(22) Anmeldetag: 09.02.2007
(51) Int. Cl.: A61K 9/20

(54) **PHARMAZEUTISCHE ZUBEREITUNG AUF BASIS VON MIKROTABLETTEN**
MICROTABLET-BASED PHARMACEUTICAL PREPARATION
PREPARATION PHARMACEUTIQUE A BASE DE MICROCOMPRIMES

(30) Priorität: 10.02.2006 DE 102006006532
(43) Veröffentlichungstag der Anmeldung: 05.11.2008
(73) Patentinhaber: Biogenerics Pharma GmbH, 20249 Hamburg (DE)
(72) Erfinder: RÖHR, Wolfgang, 20249 Hamburg (DE)
(74) Vertreter: Mann, Volker
(86) Internationale Anmeldenummer: PCT/DE2007/000415
(87) Internationale Veröffentlichungsnummer: WO 2007/090393

(56) Entgegenhaltungen:
- WO-A-00/67695
- WO-A-02/066025
- WO-A-03/074034
- DE-A1- 19 927 689
- GB-A- 1 275 706
- Dr. rer. nat Paul Heinz List: "Arzneiformenlehre", 1976, Wissenschaftliche Verlagsgesellschaft MBH, Stuttgart ISBN: 3-8047-0522-7 pages 55-56, * the whole document *

## Beschreibung

Die Erfindung betrifft eine neue pharmazeutische Zubereitung gemäß Anspruch 1.

Mikrotabletten sind beispielsweise aus der DE 342 26 19 A1 bekannt. In der Schrift werden zylindrische Formkörper mit konvexer Ober- und Unterseite beschrieben, deren Zylinderdurchmesser und Höhe unabhängig voneinander im Bereich von 1,0 bis 2,5 mm liegen und sich zueinander wie 1:0,5 bis 1,5 verhalten.

Es ist bekannt, mit Hilfe von Mikrotabletten verschiedene Wirkstoffe wie zum Beispiel Pantoprazol (DE 696 261 16 T 2), Analgetika (DE 199 016 83 B4), Alkylhydrogenfumarate und Omeprazol (196 260 45 C2) pharmazeutisch zu applizieren.

Die Problematik bei der pharmazeutischen Applikation von Wirkstoffen wird in der WO 00/67695 geschildert. Danach erfolgt die Applikation von Mikrotabletten üblicherweise in Kapseln. Nachteilig hierbei ist für den Patienten die schwierige und aufwendige individuelle Dosierung (WO 00/67695, Seite 1, Zeilen 19 bis 21). In dem Dokument wird weiter ausgeführt, dass die Verwendung von Hartgelatinekapseln dieses Problem nicht löst, da diese Kapsel nicht oder nur schwer von Patienten geschluckt werden können (WO 00/67695, Seite 1, Zeile 22 bis 26). Eine sichere individuelle Dosierung ist durch Entnahme und Aufteilung des Kapselinhalts praktisch nicht möglich, da die Inhalte einer oder mehrerer Kapseln auf die geforderte Menge auf die geforderte Menge aufgeteilt werden müssten. Das kann ein Patient jedoch nicht oder nur mit großem Aufwand durchführen (WO 00/67695, Seite 1, Zeilen 33 bis 37).

In der EP 1 185 253 B1 werden orale Applikationseinheiten mit den Wirkstoffen Tramadol und Diclofenac beschrieben. Die beiden Wirkstoffe sind in jeweils separat formulierten Untereinheiten in der Applikationseinheit enthalten [0007].

Bei den Untereinheiten im Sinne der EP 1 185 253 B1 handelt es sich um feste Arzneimittelformulierungen, die neben dem jeweiligen Wirkstoff und/oder seinen jeweiligen physiologisch verträglichen Salzen die üblichen Hilfs-Zusatzstoffe enthalten [0010].

Die Untereinheiten im Sinne der EP 1 185 253 B1 können in multipartikulärer Form zum Beispiel als Mikrotabletten vorliegen [0011].

Die in der EP 1 185 253 B1 beschriebene orale Applikationseinheit kann in Form eines Sachets, einer Kapsel oder einer Tablette vorliegen [0023].

Aufgabe der vorliegenden Erfindung ist eine neue pharmazeutische Zubereitung bei denen Mikrotabletten nach den Bedürfnissen des Patienten eingestellt und von dem Patienten leicht appliziert werden können.

Es wurde eine pharmazeutische Zubereitung in fließfähiger Form auf Basis von Mikrotaletten gefunden die dadurch gekennzeichnet ist, dass die pharmazeutische Zubereitung unterschiedliche Mikrotabletten mit verschiedenen Wirkstoffen enthält,
wobei alle Mikrotabletten gleiche Raumform, gleiches Gewicht und die gleiche Dichte aufweisen und
wobei die Mikrotabletten in homogener Mischung vorliegen.

Alle Mikrotabletten für die erfindungsgemäßen pharmazeutischen Zubereitungen weisen die gleiche Dichte auf und lassen sich dadurch leicht dosieren und zu individuellen Zubereitungen zusammenstellen.

Die erfindungsgemäßen Zubereitungen liegen in fließfähiger Form Auf Grund der gleichen Dichte der unterschiedlichen Mikrotabletten tritt keine Entmischung der unterschiedlichen Mikrotabletten ein, so dass sie sich ähnlich wie eine Flüssigkeit dosieren lassen.

Die erfindungsgemäßen Zubereitungen können leicht von den Patienten appliziert werden.

In der vorliegenden Erfindung liegen die Mikrotabletten in der erfindungsgemäßen pharmazeutischen Zubereitung in homogener Mischung vor.

In den erfindungsgemäßen pharmazeutischen Zubereitungen sind die Inhaltsstoffe weitgehend gleichmäßig verteilt. Dadurch dass die einzelnen Mikrotabletten gleiche Dichte haben, bleibt die gleichmäßige Verteilung der Inhaltsstoffe konstant; es kann keine Auftrennung der Mikrotabletten in der erfindungsgemäßen Zubereitung durch unterschiedliche Dichte eintreten.

Die Inhaltsstoffe der Mikrotabletten können Wirkstoffe, weitere Hilfsstoffe und Füllstoffe sein.

Im Rahmen der vorliegenden Erfindung sind verschiedene Wirkstoffe oder Wirkstoffkombinationen auf verschiedene Mikrotabletten verteilt. Hierdurch ist es leicht möglich, den Wirkstoffgehalt in den einzelnen Zubereitungen zu variieren und den individuellen Bedürfnissen der einzelnen Patienten anzupassen.

Auf diese Weise ist es erfindungsgemäß möglich, die Zubereitungen einfach aus Mikrotabletten als "Bausteine" beispielsweise nach den Angaben des Arztes individuell zusammenzusetzen und in eine leicht zu applizierende Form zu bringen. Die Zusammenstellung der Zubereitung kann beispielsweise auch in der Apotheke mit dort verfügbaren üblichen Mitteln erfolgen.

Es ist selbstverständlich auch möglich im Rahmen der vorliegenden Erfindung individualisierte Standartzubereitungen zur Verfügung zu stellen.

Die Bereitstellung der erfindungsgemäßen Zubereitungen erfolgt am einfachsten durch Abwiegen der einzelnen Sorten von Mikrotabletten für eine Zubereitung.

Die Applikation der erfindungsgemäßen pharmazeutischen Zubereitungen kann einfach über einen Dosierlöffel erfolgen, da durch die gleichmäßige Verteilung der Mikrotabletten auch die Inhaltsstoffe gleichmäßig verteilt sind. Es ist daher nicht erforderlich, die Mikrotabletten in Kapseln abzupacken.

Als Wirkstoffe für die Mikrotabletten kommen grundsätzlich alle festen Wirkstoffe in/frage, die in Mikrotabletten verarbeitet werden können.

Zu den Wirkstoffen im Sinne der vorliegenden Erfindung gehören beispielsweise an sich bekannte Pharmazeutika, homoöpatisch wirkende Stoffe, Vitamine, Mineralstoffe, probiotische Wirkstoffe, Enzyme, Nahrungsergänzungsmittel und Pflanzenextrakte.

Beispielsweise seien die folgenden Wirkstoffe genannt:
Vitamine, wie beispielsweise Biotin, Choline, Cobalamin (B12), Folate, Inositol, Niacin (B3), PABA (Para-aminobenzoic Acid), Pantothenic Acid (B5), Pyridoxine (B6), Riboflavin (B2), Thiamin (B1), Tocopherols, Tocotrienols, Vitamin A, Vitamin B, Vitamin C, Vitamin D, natürliches Vitamin E, synthetisches Vitamin E und Vitamin K. Die Vitamine könne synthetischen oder natürlichen Ursprungs sein und können auch in verkapseitem Zustand vorliegen.

Mineralien, wie beispielsweise Bor, Calcium, Chlor, Chrom, Kupfer, Germanium, Iod, Eisen, Magnesium, Mangan, Mineralien (Chelated), Mineralien (Colloidal), Mineralien (Coral), Mineralien (Microencapsulated), Mineralien (Yeast), Molybden, Phosphor, Calcium, Selen, Silicon, Natrium, Vanadium, Zink.

Aminosäuren, wie beispielsweise L-Alanin, L-Arginin, L-Carnitin, L-Carnosin, L-Citrullin, L-Cystin, L-Glutamin, L-Histindin, L-Isoleucin, L-Leucin, L-Lysin, L-Methionin, L-Ornithin, L-Phenylalanin, L-Prolin, L-Serin, L-Taurin, L-Theanin, L-Tyrosin, L-Valin.

Nahrungsergänzungsmittel, wie beispielsweise 5-HTP, Acetyl-L-Carnitin, Alpha Lipoic Acid, Alpha-Caroten, Anthocyanidine, Arabinogalactan, Arabinoxylan, Arachidonic Acid, Astaxanthin, ATP (Adenosine Triphosphate), Bee Pollen, Bee Propolis, Berberine, Beta-1,3-Glucan, Beta-1,6-Glucan, Beta-Caroten, Bromelain, Carotenoide, Carrageenan, Cartilage (Bovine), Cartilage (Shark), Casein, Catechine, Cellulose, Cetyl Myristoleat, Chitosan, Chlorophyll, Chondroitin Sulfat, Citicolin, Citrus Flavonoide, CLA (Conjugated Linoleic Acid), Collagen (Bovine), Hühner-Collagen, Fisch-Collagen, Collagen (Porcine), Colostrum, CoQ10 (Coenzym Q10), Creatin, Curcumin, D-Mannose, DAG (diacylglycerol) oil, Daidzein, DHA (Docosahexaenoic Acid), DHEA, Diindolylmethan, EGCG (Epigallocatechin-3 Gallat), EPA (Eicosapentaenoic Acid), Essential Fatty Acids, Fiber, Fischöl, Flavonoide, FOS (Fructooligosaccharide), Genistein, GLA (Gamma Linoleic Acid), Glucomannan, Glucosamin HCL, Glucosamin Sulfat, Glutathion, GPC (Glycerophosphocholin), Grünlippige Muschel, Gum Arabic, Gum Guar, Hesperidin, Huperzin, Hyaluronic Acid, Immunoglobulin, Indole-3-Carbinol, Inositol, Inulin, IP-6 (Inositol Hexaphosphat), Ipriflavon, Isoflavone, Isomalt, Lactoferrin, Lactose, Lanolin, Lecithin, Lignans, Lutein, Lutein Esters, Lycopen, Maltitol, Melatonin, Microcrystaline Hydroxypitite, MSM (Methylsulfonylmethan), N-Acetyl-Cystein, N-Acetyl-Glucosamin, Octacosanol, Octopamin, Oligomeric Proanthocyanidine (OPCs), Omega-3 Fettsäure, Omega-6 EFAs, Austernschale, Papain, Pearl Powder, Pectin, Pepsin, Phosphatidylcholin, Phosphatidylserin, Phospholipide, Plant Sterol/Stanol Esters, Policosanol, Polyphenole, Eiweiß, Fischprotein, Reisprotein, Seidenprotein, Sojaprotein, Molkeprotein, Pyruvat, Quercetin, Red Yeast Rice, Resveratrol, Ribose, Royal Jelly, Rutin, Salivarius, SAM-e, Seegurke, Silymarin, isoliertes Sojaprotein, Squalene, Super Oxide Dismutase, Theobromin, Velvet Antler, Vincamin, Vinpocetin, isoliertes Molkeprotein, Xanthan, Xylitol, Zeaxanthin, Zein.

Pflanzenextrakte, wie beispielsweise Acai, Acerola, AFA (Aphanizomenon Flos Aquae), Agaricus Blazei, Alfalfa, Blau-Grüne Algen, Grüne Algen, Alisma, Allicin, Aloe Vera, Aloe (Cape), Amla, Andrographis Paniculata, Angelica, Anise, Annatto, Apfel, Arjuna, Amica, Artischocke, Ashwagandha, Asparagus Racemosus, Astragalus, Atractylodes, Avocadoöl, Bacopa, Banaba Leaf, Berberitze, Gerstengras, Basilikum, Belleric Myrobalan, Beides, Bilberry, Bitter Melon (Karela), Bitter Orange (Citrus Aurantium), Black Cohosh, Black Currant Seed, Schwarzer Pfeffer, Schwarze Walnuss, Brombeere, Blasentang, Blessed Thistle, kanadisches Blutkraut, Frauenwurzel, Blaubeere, Wasserhanf, Borretsch, Boswellia, Brokkoli, Buchu, Bupleurum, große Klette, Butcher's Broom, Pestwurz, Ringelblume, kalifornischer Mohn, Kampfer, Camu Camu, Raps, Rapsöl, Capsaicin, Kardamom, Johannisbrotbaum, Cascara Sagrada, Kassia, Rizinusöl, Cat's Claw, Katzenminze, Catuaba, Cayennepfeffer, Cereal Grasses, Kamille, Chanca Piedra, Chaparral, Mönchspfeffer (Vitex), Vogelmiere, Chicorée, Chlorella, Schokolade, Zimt, Zitrusgewächs, Nelke, Bärlapp, Cnidium, Kakao, Codonopsis, Coix, Coleus Forskohlii, Huflattich, Beinwell, Koralle, Cordyceps, Corydalis, Cramp Bark, Moosbeere, Samenextrakt der Moosbeere, Samenöl der Moosbeere, Cyperus, Damiana, Löwenzahn, Teufelskralle, Dong Quai, Rotalge, Sonnenhut, Holunderbeere, Helenenkraut, Eleuthero, Emblic, Meerträubchen, ätherische Öle, Eukalyptus, Eucommia, Nachtkerze, Evodia Rutaecarpa (Wu Zhu Yu), Augentrost, False Unicorn, Fenchel, Bockshornklee, Mutterkraut, Leinsamen, Leinsamenöl, Blütenstaub, Fo-Ti, Weihrauch, Früchte, Ganoderma, Garcinia Cambogia, Knoblauch, Enzian, Ingwer, Ginkgo Biloba, amerikanischer Ginseng, orientalischer Ginseng, kanadische Gelbwurzel, Gotu Kola, Grains, Extrakt des Weintraubenkerns, Extrakt der Weintraubenschale, Extrakt des Grapefruitsamens, Weintrauben, Graviola, Green Foods, Guacatonga, Guarana, Guggul, Gymnema Sylvestre, Weißdorn, Hanf, Hibiskus, Honig, Honey Bush, Hoodia Gordonii, Hopfen, Andom, Homy Goat Weed (Epimedium), Rosskastanie, Schachtelhalm, Ysop, Indian Long Pepper, Irisch Moos, Jiaogulan, Jojoba, Wacholder, Kava, Seetang, Kola Nut, Kudzu, Kukui Nut, Lavendel, Zitronenmelisse, Zitronengras, Lakritz/ Süßholz, Lobelie, Luo Han Guo, Maca, Magnolie, Maitake, Mangosteen, Manuka, Eibisch, Mädesüß, Melilotus Officinalis, Mariendistel, Mutterkraut, Mucuna Pruriens, Muira Puama, Königskerze, Muscadine, heilkräftige Pilze, Senf, Myrrhe, Nattokinase, Neem, Nessel, Noni, Kaktus, Nori, Nüsse, Haferflocken, Haferkörner, Haferstroh, Olive, Olivenblatt, Olivenöl, Oregano, Oregon Grape, Papaya, Petersilie, Passionsblume, Pau D'Arco, Pfingstrose, Pfefferminze, Perilla, Perilla Seed Oil, Immergrün, Pine Bark Extract, Wegerich, Pleurisy, Polygonum Cuspidatum, Granatapfel, Poria, Prickly Ash, Flohsamen, Kürbiskerne, Kürbiskernöl, Pygeum, Himbeere, Himbeerblatt, Rotklee, Öl des Samens der roten Himbeere, Rehmannia, Ling-zhi-Pilz, Rhodiola, Rhabarber, Reisklee, Rotbusch, Rose Hips, Rosemarin, Färberdistel, Färberdistelöl, Salbei, Salvia Root, Sandelbaum, Sangre de Grado, Sarsaparille, Sägerpalme, Schizandra, Sanddorn, Seegras, Samen, Sennesblätter, Sesamöl, Sesamkeme, Shea, Shepard's Purse, Shiitake, Silymarin, Skullcap, Ulme, Soja, Sojaöl, grünze Minze, Spirulina, Sprossen, Squaw Vine, Johanniskraut, Stevia, Erdbeere, Suma, Sonnenblumenöl, Sonnenblumenkeme, Tamanu Oil, Teebaum, Schwarzer Tee, Grüner Tee, Thymian, Tomato Fiber, Tongkat Ali, Tribulus Terrestris, Triphala, Tulsi (Holy Basil), Gelbwurz, Uva Ursi, Baldrian, Vanille, Gemüse, Eisenkraut, Vinca Minor, Weinessig, Wasabia Japonica, Weizenkeim, Weizengras, Weiße Bohne, White Willow Bark, Wildkirsche, Wilde Süßkartoffel, Zaubemuss, Wolfberry (Lycium), Wermut, Yacon, Schafgarbe, Hefe, Gelber Ampfer, Yerba Mate, Yerba Santa, Ylang Ylang, Yohimbe, Yucca, Zizyphus.

Die Mikrotabletten einer Zubereitung im Rahmen der vorliegenden Erfindung müssen alle das gleiche Gewicht und die gleiche Raumform haben. Die Einstellung des Gewichts nach Auswahl der Wirkstoffe durch Variation der Füllstoffe.

Bei anderen Zubereitungen können selbstverständlich Mikrotabletten mit einem anderen Gewicht und einer anderen Raumform eingesetzt werden.

Das Gewicht einer einzelnen Mikrotablette hängt von der Zusammensetzung und gegebenenfalls von der Beschichtung ab. Durch eine Änderung der Anteile der Hilfsstoffe und Füllstoffe kann erreicht werden, dass Mikrotabletten mit verschiedenen Wirkstoffen das gleiche Gewicht haben.

Die Oberfläche der Mikrotabletten kann in an sich bekannter Weise beschichtet sein. Hierdurch kann beispielsweise die Applikation erleichtert oder der Wirkstoff (z.B. gegen Magensäure) geschützt werden.

Die Raumform der Mikrotabletten kann grundsätzlich frei gewählt werden. Jedoch sollte die Raumform so gestaltet sein, dass bei einer Homogenisierung keine Behinderung der einzelnen Mikrotabletten erfolgt.

Die Mikrotabletten im Rahmen der vorliegenden Erfindung weisen bevorzugt eine zylindrische, elliptische oder kugelförmige Raumform auf.

Die Größe der Mikrotabletten in zylindrischer Form im Rahmen der vorliegenden Erfindung kann im allgemeinen eine Höhe und einen Durchmesser von jeweils etwa 1 bis etwa 4 mm, bevorzugt von 1 bis 2,5 mm aufweisen.

Beispielsweise kann eine Mikrotablette in zylindrischer Form mit einer Höhe und einem Durchmesser von jeweils etwa 2 mm ein Gewicht von 6,5 bis 8,5 mg haben.

Die Mikrotabletten im Rahmen der erfindungsgemäßen Zubereitungen sind sehr genau gearbeitet und erlauben nur sehr geringe Abweichungen im Rahmen der internationalen Pharmakopöen auf.

Die Herstellung der Mikrotabletten im Rahmen der erfindungsgemäßen Zubereitungen ist an sich bekannt.

Die Homogenisierung (d.h. die gleichmäßige Verteilung) der Mikrotabletten in einer erfindungsgemäßen Zubereitung kann nach an sich bekannten Verfahren erfolgen. Hierbei werden die Mikrotabletten nicht zerstört.

Es wurde auch ein Verfahren zur Herstellung von pharmazeutischen Zubereitungen auf Basis von Mikrotabletten mit verschiedenen Inhaltstoffen gefunden, das dadurch gekennzeichnet ist, dass Mikrotabletten mit gleicher Raumform und gleichem Gewicht homogen gemischt werden.

Die erfindungsgemäß hergestellten Zubereitungen können über lange Zeit stabil in einer Flasche aufbewahrt werden und bei der Applikation aus der Flasche in ein Messgefäss gefüllt werden. Die Zusammensetzung der Rezeptur und die Fließfähigkeit bleiben dabei erhalten.

Die erfindungsgemäßen Zubereitungen lassen sich in vorteilhafter Weise leicht herstellen und in individualisierter Form leicht applizieren. Sie erweitern in neuer Weise die Darreichungsmöglichkeiten von Mitteln zum Behandeln und Vorbeugen von Krankheiten.

### Beispiel:

Zubereitung für einen Diabetiker zur Behandlung der Krankheitssymptome: Es werden die folgenden vier Typen
Bio - fruits n Greens 2000 mg per day x 30 Tage = 60 Gramm - Antioxidants
Bio- Glucoban 350 mg per day x 30 =10.5 Gramm - Kombination von 4 Extrakten (Mormodica charantia,Legerstroemia Speciosa, Centella Asiatika und Alpha Lipoic Acid), die als Phytoinsulin, der Verbesserung der Durchblutung, zum besseren Glucosetransport in die Zellen und zur Reduzierung der Insulinresistenz wirken.
Bio- Eye Health 250 mg per day x 30 = 7.5 Gramm -Kombination aus Carotenoiden, Lutein, Astanxathin , wenn die Sehkraft schon beeinträchtigt ist.
Bio- CardioHealth - 500mg per day x 30 = 15 Gramm- Kombination aus natürlichen Tocotrienolen und Tocopherolen (Full Spektrum Vitamin E), natürlichem Vitamin C und Coenzym Q10.

Jede Mikrotabletten hat 5mg als Aktivsubstanz und hat ein Gewicht von 7.5mg incl. der Zusatzstoffe.

Die Dosierungen hängen von dem Gewicht und Krankheitsbild des Patienten ab.

Die Mikrotabletten sind zylindrisch und haben eine Höhe von 2 mm und einen Durchmesser von 2 mm.

Gemäß der vorgegeben Dosierung werden die vier verschieden Typen an Mikrotabletten in einem Schüttelbecher homogenisiert und können mit einem Meßgefäß entnommen werden. Die Zubereitung ist gut fließfähig.

## Patentansprüche

1. Pharmazeutische Zubereitung in fließfähiger Form auf Basis von Mikrotabletten, **dadurch gekennzeichnet, dass** die pharmazeutische Zubereitung unterschiedliche Mikrotabletten mit verschiedenen Wirkstoffen enthält,
wobei alle Mikrotabletten gleiche Raumform, gleiches Gewicht und die gleiche Dichte aufweisen und
wobei die Mikrotabletten in homogener Mischung vorliegen.

2. Pharmazeutische Zubereitung nach Anspruch 1, **dadurch gekennzeichnet, dass** sich die unterschiedlichen Mikrotabletten bei der Dosierung nicht entmischen.

3. Pharmazeutische Zubereitung nach einem der Ansprüche 1 oder 2, **dadurch gekennzeichnet, dass** als Inhaltsstoffe der Mikrotabletten pharmazeutische Wirkstoffe, Hilfsstoffe und Füllstoffe eingesetzt werden.

4. Pharmazeutische Zubereitung nach einem der Ansprüche 1 bis 3, **dadurch gekennzeichnet, dass** die Mikrotabletten eine zylindrische, elliptische oder kugelförmige Raumform aufweisen.

5. Pharmazeutische Zubereitung nach einem der Ansprüche 1 bis 4, **dadurch gekennzeichnet, dass** Mikrotabletten eine zylindrische Form mit einer Höhe von 1 bis 3 mm und einem Durchmesser von 1 bis 3 mm aufweisen.

6. Pharmazeutische Zubereitung nach einem der Ansprüche 1 bis 5, **dadurch gekennzeichnet, dass** die Mikrotabletten ein Gewicht im Bereich von 4 bis 8 mg aufweist.

7. Verfahren zur Herstellung von pharmazeutischen Zubereitungen nach den Ansprüchen 1 bis 6, **dadurch gekennzeichnet, dass** die unterschiedlichen Mikrotabletten homogen gemischt werden.

## Claims

1. Pharmaceutical preparation in flowable form based on microtablets, **characterized in that** the pharmaceutical preparation contains various microtablets with different active compounds,
all the microtablets having the same spatial shape, the same weight and the same density and the microtablets being present in a homogeneous mixture.

2. Pharmaceutical preparation according to Claim 1, **characterized in that** the various microtablets do not separate during dosage.

3. Pharmaceutical preparation according to one of Claims 1 or 2, **characterized in that** pharmaceutical active compounds, auxiliary substances and fillers are employed as constituents of the microtablets.

4. Pharmaceutical preparation according to one of Claims 1 to 3, **characterized in that** the microtablets have a cylindrical, elliptical or spherical spatial shape.

5. Pharmaceutical preparation according to one of Claims 1 to 4, **characterized in that** the microtablets have a cylindrical shape with a height of from 1 to 3 mm and a diameter of from 1 to 3 mm.

6. Pharmaceutical preparation according to one of Claims 1 to 5, **characterized in that** the microtablets have a weight in the range of from 4 to 8 mg.

7. Process for the preparation of pharmaceutical preparations according to Claims 1 to 6, **characterized in that** the various microtablets are mixed homogeneously.

## Revendications

1. Préparation pharmaceutique sous une forme pouvant s'écouler à base de microcomprimés, **caractérisée en ce que** la préparation pharmaceutique contient différents microcomprimés contenant différentes actives,
tous les microcomprimés présentant la même forme spatiale, le même poids et la même densité, et
les microcomprimés se trouvant en mélange homogène.

2. Préparation pharmaceutique selon la revendication 1, **caractérisée en ce que** le mélange des différents microcomprimés ne se sépare pas lors du dosage.

3. Préparation pharmaceutique selon l'une quelconque des revendications 1 ou 2, **caractérisée en ce que** des actives pharmaceutiques, des adjuvants et des charges sont utilisés comme constituants des microcomprimés.

4. Préparation pharmaceutique selon l'une quelconque des revendications 1 à 3, **caractérisée en ce que** les microcomprimés présentent une forme spatiale cylindrique, elliptique ou sphérique.

5. Préparation pharmaceutique selon l'une quelconque des revendications 1 à 4, **caractérisée en ce que** les microcomprimés présentant une forme cylindrique d'une hauteur de 1 à 3 mm et d'un diamètre de 1 à 3 mm.

6. Préparation pharmaceutique selon l'une quelconque des revendications 1 à 5, **caractérisée en ce que** les microcomprimés présentent un poids dans la plage de 4 à 8 mg.

7. Procédé pour la préparation de préparations pharmaceutiques selon les revendications 1 à 6, **caractérisé en ce que** les différents microcomprimés sont mélangés de manière homogène.
